(19) Europäisches Patentamt
European Patent Office
Office européen des brevets

(11) Veröffentlichungsnummer: **0 013 893**
**A1**

(12) # EUROPÄISCHE PATENTANMELDUNG

(21) Anmeldenummer: **80100061.3**

(22) Anmeldetag: **07.01.80**

(51) Int. Cl.³: **C 07 D 307/12**, C 07 D 307/16, C 07 D 307/24, C 07 D 307/94 // C07D321/08

(30) Priorität: **17.01.79 DE 2901644**

(43) Veröffentlichungstag der Anmeldung: **06.08.80 Patentblatt 80/16**

(84) Benannte Vertragsstaaten: **BE CH DE FR GB IT NL**

(71) Anmelder: **BAYER AG, Zentralbereich Patente, Marken und Lizenzen, D-5090 Leverkusen 1, Bayerwerk (DE)**

(72) Erfinder: **Scharf, Hans-Dieter, Prof. Dr., Greppstrasse 15 a, D-5106 Roetgen (DE)**
Erfinder: **Frauenrath, Herbert, Haarhofstrasse 72, D-5100 Aachen (DE)**

(54) **Verfahren zur Herstellung von Tetrahydrofuran-3-aldehyden.**

(57) Die Erfindung betrifft ein neues Verfahren zur Herstellung von Tetrahydrofuran-3-aldehyden der Formel (I)

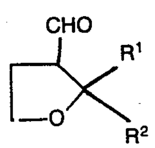

(I)

in welcher $R^1$ und $R^2$ die in der Beschreibung angegebene Bedeutung haben, das dadurch gekennzeichnet ist, daß man 4,5-Dihydro-1,3 dioxepine der Formel (II)

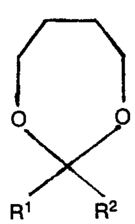

(II)

mit katalytischen Mengen o-Tolylsäure auf Temperaturen zwischen 100 und 250° C erhitzt.

EP 0 013 893 A1

0013893

BAYER AKTIENGESELLSCHAFT
Zentralbereich
Patente, Marken und Lizenzen

5090 Leverkusen, Bayerwerk
Rt-by
Typ IVa

Verfahren zur Herstellung von Tetrahydrofuran-3-aldehyden

Die Erfindung betrifft ein neues Verfahren zur Herstellung von Tetrahydrofuran-3-aldehyden.

Es ist bekannt, daß 4,7-Dihydro-1,3-dioxepine unter Basenkatalyse zu 4,5-Dihydro-1,3-dioxepinen isomerisieren. Aus 2-(4-Methoxy-phenyl)-4,7-dihydro-1,3-dioxepin wird jedoch unter den üblichen Reaktionsbedingungen - Erhitzen mit Kalium-tert.-butylat in tert.-Butanol - nicht 2-(4-Methoxy-phenyl)-4,5-dihydro-1,3-dioxepin, sondern 2-(4-Methoxy-phenyl)-tetrahydrofuran-3-aldehyd gebildet. Experimentelle Details und Produkteigenschaften hierzu sind nicht bekannt (vgl. C. R. Acad. Sci Ser. C 273, (1971), 1655-1657).

Es wurde nun gefunden, daß man die Tetrahydrofuran-3-aldehyde der Formel (I)



BAD ORIGINAL

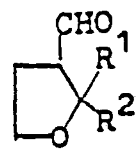

(I)

in welcher

$R^1$ für Wasserstoff, Alkyl oder gegebenenfalls substituiertes Phenyl steht und

$R^2$ für gegebenenfalls substituiertes Alkyl, $C_{1-4}$-Alkoxycarbonyl, $C_{1-4}$-Alkoxycarbonylmethylen, gegebenenfalls substituiertes Phenyl oder zusammen mit $R^1$ für Alkandiyl steht,

in hohen Ausbeuten erhält, wenn man 4,5-Dihydro-1,3-dioxepine der Formel (II)

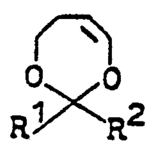

(II)

in welcher

$R^1$ und $R^2$ die oben angegebene Bedeutung haben,

mit katalytischen Mengen o-Tolylsäure auf Temperaturen zwischen 100 und 250°C erhitzt.

Überraschenderweise können nach dem erfindungsgemäßen Verfahren Tetrahydrofuran-3-aldehyde durch säurekatalysierte Isomerisierung von 4,5-Dihydro-1,3-dioxepinen in sehr guten Ausbeuten hergestellt werden. Dies war nach dem Stand der Technik zu erwarten.

Die nach dem erfindungsgemäßen Verfahren erhältlichen Tetrahydrofuran-3-aldehyde sind zum Teil neu. Sie weisen starke insektizide und akarizide Eigenschaften auf.

Gegenstand der Erfindung ist vorzugsweise die Herstellung von Verbindungen der Formel (I), in welcher

$R^1$ für Wasserstoff, geradkettiges wie auch verzweigtes Alkyl mit 1 bis 4 Kohlenstoffatomen oder Phenyl steht und

$R^2$ für geradkettiges oder verzweigtes Alkyl mit 1 bis 4 Kohlenstoffatomen, Methoxy- oder Äthoxycarbonyl, Methoxy-oder Äthoxycarbonylmethylen, für gegebenenfalls durch $C_{1-4}$-Alkoxy substituiertes Phenyl oder zusammen mit $R^1$ für geradkettiges oder verzweigtes $\alpha,\omega$-Alkandiyl mit 3 bis 9 Kohlenstoffatomen steht.

Verwendet man als Ausgangsverbindung beispielsweise 2-Äthyl-2-phenyl-4,5-dihydro-1,3-dioxepin, so kann die erfindungsgemäße Reaktion durch folgendes Formelschema wiedergegeben werden:

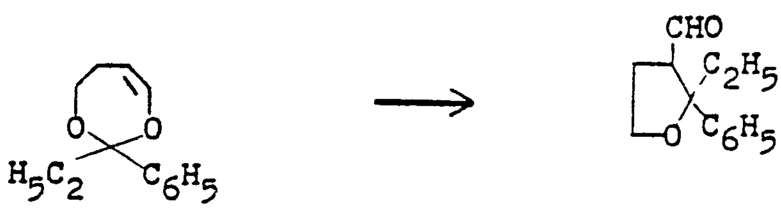

Die als Ausgangsstoffe zu verwendenden 4,5-Dihydro-1,3-dioxepine sind durch Formel (II) definiert.

Vorzugsweise stehen darin $R^1$ und $R^2$ für diejenigen Reste, welche bei der Definition der Reste $R^1$ und $R^2$ in Formel (I) als bevorzugt genannt wurden.

Als Beispiele für die Ausgangsverbindungen der Formel (II) seien genannt:

2-Methyl-, 2-Äthyl-, 2-Phenyl-, 2,2-Diphenyl-, 2-(4-Methoxyphenyl)-, 2-Methyl-2-carbethoxi-, 2-Methyl-2-carbethoxymethylen-, 2,2-Dimethyl-, 2,2,-Diäthyl-, 2-Methyl-2-äthyl-, 2-Methyl-2-phenyl- und 2-Äthyl-2-phenyl-4,5-dihydro-1,3-dioxepin sowie 7,12-Dioxaspiro /5,6/ dodec-(8)-en.

Die 4,5-Dihydro-1,3-dioxepine der Formel (II) sind teilweise bekannt (vgl. C.R. Acad. Sci. Ser. C 273 (1971), 1655-1657). Man erhält sie durch basenkatalysierte Isomerisierung von 4,7-Dihydro-1,3-dioxepinen der Formel (III)

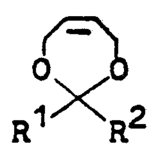

(III)

in welcher

$R^1$ und $R^2$ die oben angegebene Bedeutung haben,

im allgemeinen durch Erhitzen in Gegenwart von 0,1 bis 2, vorzugsweise 0,1 bis 1,5 Moläquivalenten einer Base wie z.B. Kalium-tert.-butylat und gegebenenfalls unter Verwendung eines Lösungsmittels oder Verdünnungsmittels wie z.B. tert.-Butanol oder Dimethylsulfoxid auf Temperaturen zwischen 20 und 200°C, vorzugsweise zwischen 50 und 150°C. Die Aufarbeitung kann auf übliche Weise erfolgen, beispielsweise indem man das Reaktionsgemisch nach Abkühlen in Eiswasser gießt, mit einem mit Wasser nicht mischbaren Lösungsmittel wie z.B. Diäthyläther extrahiert, die organische Phase wäscht und trocknet, nach Filtration des Lösungsmittels abdestilliert und das zurückbleibende Produkt durch Destillation reinigt.

Die 4,7-Dihydro-1,3-dioxepine der Formel (III) sind bekannt (vgl. Bull Soc. Chim. France 1975, 1763-1766 und 2558-2560; US-PS 3 116 298) oder lassen sich analog zu bekannten Verfahren herstellen.

Als Ausgangsverbindungen bevorzugt sind Verbindungen der Formel (III), in welcher $R^1$ und $R^2$ die bei der Definition der Reste $R^1$ und $R^2$ in Formel (I) als bevorzugt angegebene Bedeutung haben.

Als Beispiele seien genannt:

2-Methyl-, 2-Äthyl-, 2-Phenyl-, 2,2-Diphenyl-, 2-Methyl-2-carbethoxy-, 2-Methyl-2-carbethoxymethylen-, 2-(4-Methoxy-phenyl)-, 2,2-Dimethyl-, 2,2-Diäthyl-, 2-Methyl-2-äthyl-, 2-Methyl-2-phenyl und 2-Äthyl-2-phenyl-4,7-dihydro-1,3-dioxepin sowie 7,12-Dioxaspiso [5,6] docec-(9)-en.

Bei der Durchführung des erfindungsgemäßen Verfahrens setzt man auf 1 Mol 4,5-Dihydro-1,3-dioxepin 0,01 bis 0,2 Mol, vorzugsweise 0,05 bis 0,15 Mol o-Tolylsäure als Katalysator ein. Die Reaktionskomponenten werden im allgemeinen bei Raumtemperatur zusammen gegeben und mehrere Stunden auf eine Temperatur zwischen 100 und 250°C, vorzugsweise zwischen 120 und 200°C erhitzt. Die Umsetzung wird bei Normaldruck oder leicht erhöhtem Druck, im allgemeinen bei 1 bis 10 bar durchgeführt. Nach Reaktionsende werden die Tetrahydrofuran-3-aldehyde aus dem Reaktionsgemisch abdestilliert und gegebenenfalls durch erneute fraktionierte Destillation gereinigt. Zur Charakterisierung der Produkte dient der Brechungsindex.

## Herstellungsbeispiele

### Beispiel 1

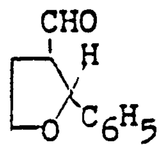

44 g (0,25 Mol) 2-Phenyl-4,5-dihydro-1,3-dioxepin werden mit 3 g o-Tolylsäure versetzt und 3 Stunden unter Rühren auf 150 °C erhitzt. Anschließend wird der entstandene 2-Phenyl-tetrahydrofuran-3-aldehyd (Mischung aus den cis- und trans-Isomeren) unter vermindertem Druck destilliert.

Ausbeute: 33,9 g (77 % der Theorie); farblose Flüssigkeit; Siedepunkt 81 °C/0,001 Torr; Brechungsindex $n_D^{20}$ 1,5395

Analog Beispiel 1 können die folgenden Verbindungen der Formel (I)

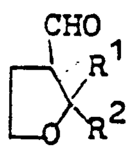

(I)

hergestellt werden.

| Beispiel Nr. | $R^1$ | $R^2$ | Ausbeute (% d.Th.) | Brechungsindex ($n_D^{20}$) | Siedepunkt (°C/Torr) |
|---|---|---|---|---|---|
| 2 | H | $p-C_6H_4-OCH_3$ | 70 | 1,5498 | 114-116/0,001 |
| 3 | $CH_3$ | $CH_3$ | 95 | 1,4418 | 58,5-60/12 |
| 4 | $CH_3$ | $C_2H_5$ | 90 | 1,4490 | 75-76,5/15 |
| 5 | $CH_3$ | $C_6H_5$ | 92 | 1,5410 | 88-90/0,5 |
| 6 | $-CH_2CH_2CH_2CH_2CH_2-$ | | 95 | 1,4840 | 122,5-123,5/15 |
| 7 | H | $CH_3$ | 15 | cis: 1,4425<br>trans: 1,4450 | cis: 82-83/100<br>trans: 75-80/100 |
| 8 | $C_6H_5$ | $C_6H_5$ | | | |
| 9 | $CH_3$ | $-CO_2C_2H_5$ | | | |
| 10 | $CH_3$ | $-CH_2-COOC_2H_5$ | | | |

Die als Ausgangsverbindungen zu verwendenden 4,5-Dihydro-1,3-dioxepine können wie folgt hergestellt werden:

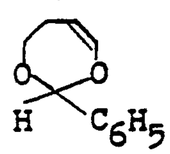

in einem 1 l -Dreihalskolben mit Rührer und Rückflußkühler mit Trockenrohr läßt man zu einer Aufschlämmung von 67,3 g (0,6 Mol) Kalium-tert.-butylat in 300 ml absol. Dimethylsulfoxid innerhalb einer Stunde 88,1 g (0,5 Mol) 2-Phenyl-4,7-dihydro-1,3-dioxepin zugetropft. Nach dem Abklingen der exothermen Reaktion wird unter Rühren 48 Stunden auf 80°C erhitzt.

Man läßt dann auf Raumtemperatur abkühlen und gießt das dunkelbraune Reaktionsgemisch unter Rühren in 500 ml Eiswasser. Zur Extraktion der organischen Phase wird die wäßrige Phase zunächst mit 500 ml, dann mehrfach mit jeweils 100 ml Diäthyläther ausgeschüttelt. Die vereinigten Ätherphasen werden dreimal mit 200 ml gesättigter Natriumchloridlösung gewaschen und mit wasserfreiem Kaliumcarbonat getrocknet. Nach dem Abfiltrieren des Trockenmittels wird das Lösungsmittel abdestilliert und das Rohprodukt durch Destillation gereinigt. Man erhält als farblose Flüssigkeit 51,1 g (58 % der Theorie) 2-Phenyl-4,5-dihydro-1,3-dioxepin vom Siedepunkt 63-64,5 $^{o}$C/0,001 Torr und dem Brechungsindex $n_D^{20}$ 1,5470.

Analog Beispiel a können die folgenden Verbindungen der Formel (II)

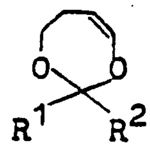

(II)

hergestellt werden.

| Bei-spiel Nr. | $R^1$ | $R^2$ | Ausbeute (% der Theorie) | Brechungs-index ($n_D^{20}$) | Siedepunkt (°C/Torr) |
|---|---|---|---|---|---|
| b | H | $p-C_6H_4-OCH_3$ | 67 | 1,5600 | 86-87/0,01 |
| c | $CH_3$ | $CH_3$ | 60 | 1,4440 | 33/34/12 |
| d | $CH_3$ | $C_2H_5$ | 66 | 1,4475 | 53,5-55/15 |
| e | $CH_3$ | $C_6H_5$ | 72 | 1,5295 | 57,5-58,5/0,001 |
| f) | $-CH_2CH_2CH_2CH_2CH_2-$ | | 72 | 1,4843 | 95,5-96/15 |

Patentanspruch

Verfahren zur Herstellung von Tetrahydrofuran-3-aldehyden der Formel (I)

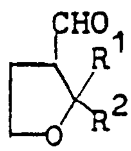

(I)

in welcher

$R^1$ für Wasserstoff, Alkyl oder gegebenenfalls substituiertes Phenyl steht und

$R^2$ für gegebenenfalls substituiertes Alkyl, $C_{1-4}$-Alkoxycarbonyl, $C_{1-4}$-Alkoxycarbonylmethylen, gegebenenfalls substituiertes Phenyl oder zusammen mit $R^1$ für Alkandiyl steht,

dadurch gekennzeichnet, daß man 4,5-Dihydro-1,3-dioxepine der Formel (II)

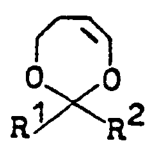

(II)

in welcher

$R^1$ und $R^2$ die oben angegebene Bedeutung haben,

mit katalytischen Mengen o-Tolylsäure auf Temperaturen zwischen 100 und 250°C erhitzt.

0013893

Europäisches Patentamt

**EUROPÄISCHER RECHERCHENBERICHT**

Nummer der Anmeldung
EP 80 10 0061

| EINSCHLÄGIGE DOKUMENTE | | | KLASSIFIKATION DER ANMELDUNG (Int Cl. ³) |
|---|---|---|---|
| Kategorie | Kennzeichnung des Dokuments mit Angabe, soweit erforderlich, der maßgeblichen Teile | betrifft Anspruch | |
| D,X | COMPTES RENDUS HEBDOMADAIRES DES SEANCES DE L'ACADEMIE DES SCIENCES, Tome 273, Nr. 23, Serie C, 8. Dezember 1971, Seiten 1655-1657 Paris, FR. VU MOC THUY: "Sur la préparation des dihydro-6,7 dioxépines-1,3, acétals énoliques cycliques"<br>* Insgesamt *<br>---- | Einziger | C 07 D 307/12<br>307/16<br>307/24<br>307/94//<br>C 07 D 321/08 |

RECHERCHIERTE SACHGEBIETE (Int Cl ³)

C 07 D 307/12
307/16
307/24
307/94

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 16-04-1980 | ALLARD |